# EUROPEAN PATENT SPECIFICATION

(11) **EP 4 472 514 B1**
(45) Date of publication and mention of the grant of the patent: **12.03.2025**
(21) Application number: 23729654.6
(22) Date of filing: 10.05.2023
(51) Int. Cl.: A61B 6/03, A61B 6/00

(54) **DUAL SOURCE CT MACHINE AND DRUM FOR DUAL SOURCE CT MACHINE**
CT-GERÄT MIT ZWEI STRAHLUNGSQUELLEN UND TROMMEL FÜR CT-GERÄT MIT ZWEI STRAHLUNGSQUELLEN
MACHINE DE TOMODENSITOMÉTRIE À DOUBLE SOURCE ET TAMBOUR POUR MACHINE DE TOMODENSITOMÉTRIE À DOUBLE SOURCE

(30) Priority: 30.05.2022 CN 202221397178 U
(43) Date of publication of application: 11.12.2024
(73) Proprietor: Siemens Shanghai Medical Equipment Ltd., Shanghai 201318 (CN)
(72) Inventor: FAN, Li Mei, Shanghai 201318 (CN); ZHU, Tie Biao, Shanghai 201317 (CN); ZHENG, Jing Ming, Shanghai 200129 (CN)
(74) Representative: Horn Kleimann Waitzhofer Schmid-Dreyer Patent- und Rechtsanwälte PartG mbB
(86) International application number: PCT/CN2023/093294
(87) International publication number: WO 2023/231729

(56) References cited:
- US-A1- 2016 058 398
- BESSON G M: "A new CT system architecture for high temporal resolution with applications to improved geometric dose efficiency and sparse sampling", PROGRESS IN BIOMEDICAL OPTICS AND IMAGING, SPIE - INTERNATIONAL SOCIETY FOR OPTICAL ENGINEERING, BELLINGHAM, WA, US, vol. 9412, 18 March 2015 (2015-03-18), pages 94120Y - 94120Y, XP060050938, ISSN: 1605-7422, ISBN: 978-1-5106-0027-0, DOI: 10.1117/12.2076222

## Description

### TECHNICAL FIELD

The invention generally relates to the technical field of medical devices, and more specifically, to a dual source CT machine and a drum for a dual source CT machine.

### BACKGROUND

A drum is a vital structural component on a CT machine, which needs to support various vital electrical components such as an X-ray tube, a detector, a high voltage generator, a high pressure tank, and a tube cooling system. In a traditional design, one CT machine is equipped with one drum, and for each type of electrical component, one electrical component is mounted to the drum. Therefore, when the CT machine scans once, the drum needs to rotate by one circle. For detection of cardiac organs, the scan needs to be completed in a short period of time. Therefore, the drum is required to have an extremely high rotational speed, and patients need to hold their breath to complete the scan.

In a traditional dual source CT machine, the drum has a cylindrical structure, and a mounting surface of each type of electrical component is on a side wall of the cylinder. Therefore, the side wall is required to have a sufficient height and thickness. Therefore, the drum with the structure has a large external dimension, and has a large weight.

For example, G.M. Besson: "A new CT system architecture for high temporal resolution with applications to improved geometric dose efficiency and sparse sampling", PROGRESS IN BIOMEDICAL OPTICS AND IMAGING, SPIE - INTERNATIONAL SOCIETY FOR OPTICAL ENGINEERING, BELLINGHAM, WA, US, vol. 9412, 18 March 2015 (2015-03-18), pages 94120Y - 94120Y, XP060050938, ISSN: 1605-7422, ISBN: 978-1-5106-0027-0, DOI: 10.1117/12.2076222 and US 2016/0058398 A1 disclose aspects of CT apparatuses.

### SUMMARY

In view of the above, in order to eliminate or alleviate the above problems, the invention discloses a drum for a dual source CT machine. The dual source CT machine using the drum is particularly suitable for heart scan, and does not require patients to hold their breath during the scan, and can perform high quality imaging in any heart rate condition. The invention further discloses a dual source CT machine using the above drum.

In the drum for a dual source CT machine of the invention, the drum is mounted to an outer rim of a bearing, and an inner rim of the bearing is mounted to a gantry of the dual source CT machine. The drum includes: a ring-shaped front drum, where the ring-shaped front drum has a first side surface and a second side surface opposite to each other, a first system mounting area configured for a first X-ray tube system to be mounted and a second system mounting area configured for a second X-ray tube system to be mounted are arranged on the first side surface of the front drum, and a front flange extending in an axial direction from an inner ring edge of the front drum is formed on the second side surface of the front drum; and a ring-shaped rear drum, where the rear drum has a first side surface and a second side surface opposite to each other, a first component mounting area configured for a first set of components matching the first X-ray tube system to be mounted and a second component mounting area configured for a second set of components matching the second X-ray tube system to be mounted are arranged on the first side surface of the rear drum, and a rear flange extending in the axial direction from an inner ring edge of the rear drum is formed on the second side surface of the rear drum. The front drum and the rear drum are arranged coaxially, the front flange and the rear flange face each other, the front drum is connected to the bearing through the front flange, and the rear drum is connected to the bearing through the rear flange.

Further, the first system mounting area includes a first tube mounting area configured for a first tube to be mounted and a first detector mounting area configured for a first detector to be mounted, and a first X-ray path is formed between the first tube and the first detector; the second system mounting area includes a second tube mounting area configured for a second tube to be mounted and a second detector mounting area configured for a second detector to be mounted, and a second X-ray path is formed between the second tube and the second detector; and the first X-ray path and the second X-ray path are both perpendicular to the axial direction, and an included angle between the first X-ray path and the second X-ray path is between 45 degrees and 135 degrees.

Further, the front drum includes at least one front protrusion protruding from the first side surface of the front drum, and the at least one front protrusion is arranged around and in contact with at least one X-ray tube system of the first X-ray tube system and the second X-ray tube system; and/or the rear drum includes at least one rear protrusion protruding from the first side surface of the rear drum, and the at least one rear protrusion is arranged around and in contact with at least one set of components of the first set of components and the second set of components.

Further, at least one of the at least one front protrusion is arranged on a radial outer side of the at least one X-ray tube system; and/or at least one of the at least one rear protrusion is arranged on a radial outer side of the at least one set of components.

Further, each of the first tube mounting area and the second tube mounting area has a through hole, so that the first tube mounted in the first tube mounting area and the second tube mounted in the second tube mounting area pass through the corresponding through holes and are mounted to the front drum.

Further, a balance weight is mounted at a position on the second side surface of the front drum staggered from the first system mounting area and the second system mounting area located on the first side surface of the front drum.

Further, one or more grooves are arranged on circumferential surfaces of inner holes of the front drum and the rear drum, and the first X-ray tube system and the second X-ray tube system are respectively connected to the first set of components and the second set of components through cables extending through the one or more grooves.

Further, each of the first component mounting area and the second component mounting area includes a high voltage generator mounting area configured for a high voltage generator to be mounted, a high pressure tank mounting area configured for a high pressure tank to be mounted, and a tube cooling system mounting area configured for a tube cooling system to be mounted, and the first component mounting area and the second component mounting area are arranged symmetrically with respect to a center of the rear drum.

Further, a heat sink and/or a heat dissipation hole is arranged on the front drum and/or the rear drum.

Further, a balance weight is mounted at a position on the second side surface of the rear drum staggered from the first component mounting area and the second component mounting area located on the first side surface of the rear drum.

According to another aspect of the invention, a dual source CT machine includes the above drum.

Through the drum of the invention and the dual source CT machine including the drum, the scan time of the CT machine can be reduced, the scan efficiency can be improved, and clearer scanned images can be obtained. In addition, the drum of the invention has a light weight and low costs, is integrated with many functions, and has a more optimized structure.

### BRIEF DESCRIPTION OF DRAWINGS

Preferred embodiments of the invention are described in detail below with reference to the drawings, so as to make a person of ordinary skill in the art be more aware of the above and other features and advantages of the invention. In the figures:
FIG. 1 shows a drum for a dual source CT machine according to an embodiment of the invention;
FIG. 2 is a side view showing a front drum of the drum in FIG. 1;
FIG. 3 is a three-dimensional view showing the front drum of the drum in FIG. 1, showing a first side of the front drum;
FIG. 4 is a three-dimensional view showing the front drum of the drum in FIG. 1 from another perspective, showing a second side of the front drum;
FIG. 5 is a side view showing a rear drum of the drum in FIG. 1;
FIG. 6 is a three-dimensional view showing the rear drum of the drum in FIG. 1, showing a first side of the rear drum; and
FIG. 7 is a three-dimensional view showing the rear drum of the drum in FIG. 1 from another perspective, showing a rear side of the rear drum.

### Reference numerals:

- 100: Drum
- 1: Front drum
- 1a: First side surface of front drum
- 1b: Second side surface of front drum
- 1c: Side end surface of front drum
- 10: Front protrusion
- 11: First system mounting area
- 111: First tube mounting area
- 112: First detector mounting area
- 12: Second system mounting area
- 121: Second tube mounting area
- 122: Second detector mounting area
- 13: Front flange
- 2: Rear drum
- 2a: First side surface of rear drum
- 2b: Second side surface of rear drum
- 2c: Side end surface of rear drum
- 20: Rear protrusion
- 21: First component mounting area
- 211: High voltage generator mounting area
- 212: High pressure tank mounting area
- 213: tube cooling system mounting area
- 22: Second component mounting area
- 221: High voltage generator mounting area
- 222: High pressure tank mounting area
- 223: Tube cooling system mounting area
- 23: Rear flange
- R1: First X-ray path
- R2: Second X-ray path
- α: Included angle
- G1: Groove on front drum
- G2: Groove on rear drum
- P1: Balance weight mounting position on front drum
- P2: Balance weight mounting position on rear drum

### DETAILED DESCRIPTION

In order to make the purposes, technical solutions, and advantages of the invention clearer, the following embodiments are provided to describe the invention in further detail.

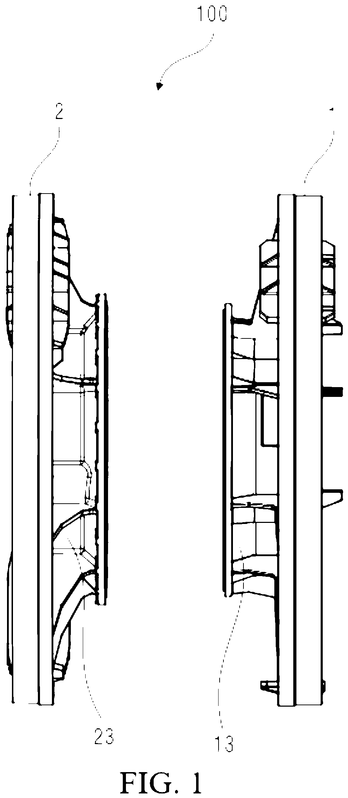

Referring to FIG. 1 to FIG. 7, the invention provides a drum 100 for a dual source CT machine. The drum 100 is mounted to an outer rim of a bearing, and an inner rim of the bearing is mounted to a gantry of the dual source CT machine. Generally, the drum 100 may include a ring-shaped front drum 1 and a ring-shaped rear drum 2. The front drum 1 and the rear drum 2 both may be made by casting aluminum. Such a casting has materials with a small weight and high strength.

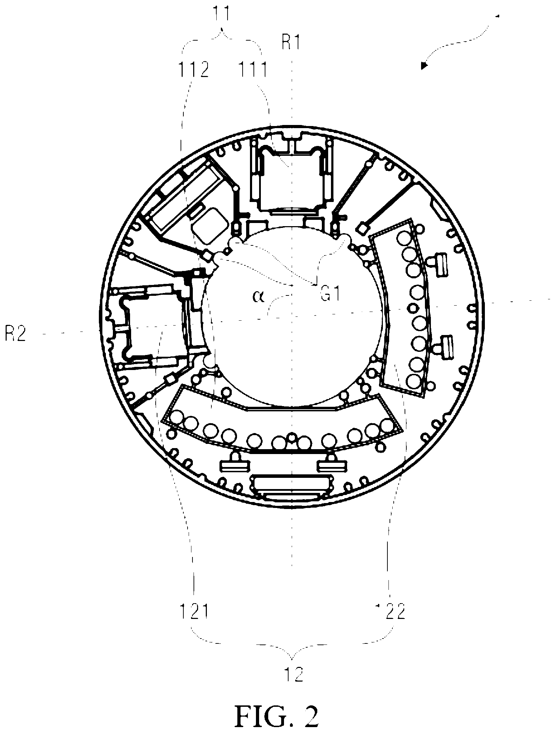

Specifically, referring to FIG. 2 to FIG. 4, the front drum 1 has a first side surface 1a and a second side surface 1b opposite to each other. A first system mounting area 11 configured for a first X-ray tube system to be mounted and a second system mounting area 12 configured for a second X-ray tube system to be mounted are arranged on the first side surface 1a of the front drum 1. A front flange 13 extending in an axial direction (the axial direction herein is an axial direction of the drum, and since the drum includes the front drum 1 and the rear drum 2 arranged coaxially, the axial direction of the drum, in fact, an axial direction of the front drum, and an axial direction of the rear drum are the same) from an inner ring edge of the front drum 1 is formed on the second side surface 1b of the front drum 1.

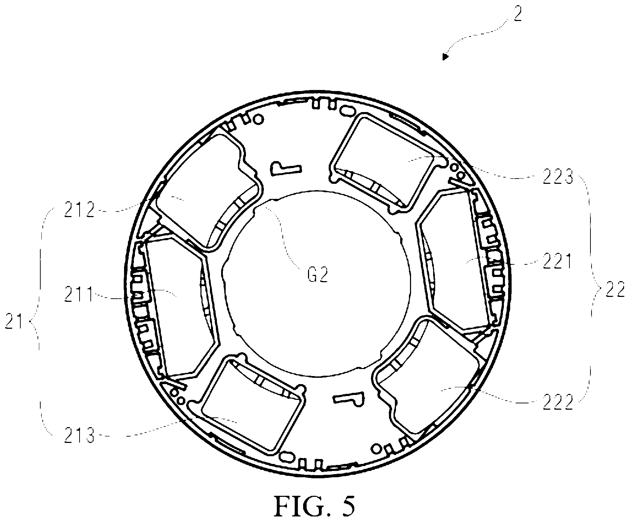

Specifically, referring to FIG. 5 to FIG. 7, the rear drum 2 has a first side surface 2a and a second side surface 2b opposite to each other. A first component mounting area 21 configured for a first set of components matching the first X-ray tube system to be mounted and a second component mounting area 22 configured for a second set of components matching the second X-ray tube system to be mounted are arranged on the first side surface 2a of the rear drum 2. A rear flange 23 extending in the axial direction from an inner ring edge of the rear drum 2 is formed on the second side surface 2b of the rear drum 2.

The front drum 1 and the rear drum 2 are arranged coaxially, and the front flange 13 and the rear flange 23 face each other. The front drum 1 is connected to the bearing through the front flange 13, and the rear drum 2 is connected to the same bearing through the rear flange 23. Specifically, the front flange 13 and the rear flange 23 may be connected to the bearing through bolts, for example.

Referring to FIG. 2, the first system mounting area 11 includes a first tube mounting area 111 configured for a first tube to be mounted and a first detector mounting area 112 configured for a first detector to be mounted. A first X-ray path R1 is formed between the first tube and the first detector. The first tube and the first detector are not shown in the figure, and only the first X-ray path R1 is schematically shown with a broken line. The second system mounting area 12 includes a second tube mounting area 121 configured for a second tube to be mounted and a second detector mounting area 122 configured for a second detector to be mounted. A second X-ray path R2 is formed between the second tube and the second detector. The second tube and the second detector are not shown in the figure, and only the second X-ray path R2 is schematically shown with a broken line. The first X-ray path R1 and the second X-ray path R2 are both perpendicular to the axial direction (that is, in a radial direction of the drum), and an included angle α between the first X-ray path R1 and the second X-ray path R2 is between 45 degrees and 135 degrees. Preferably, the included angle α is 90 degrees.

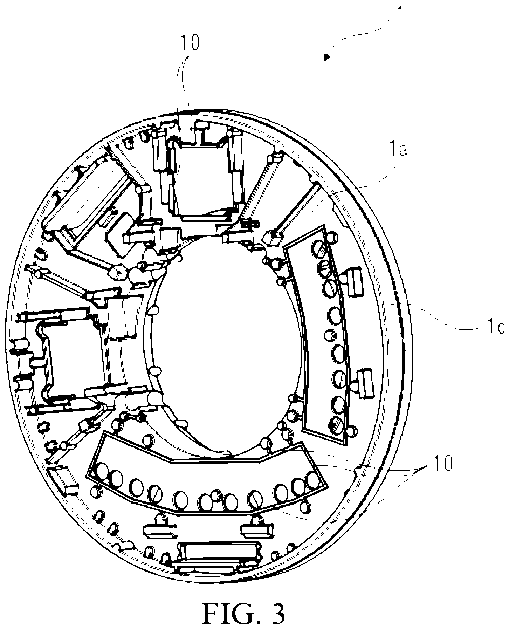

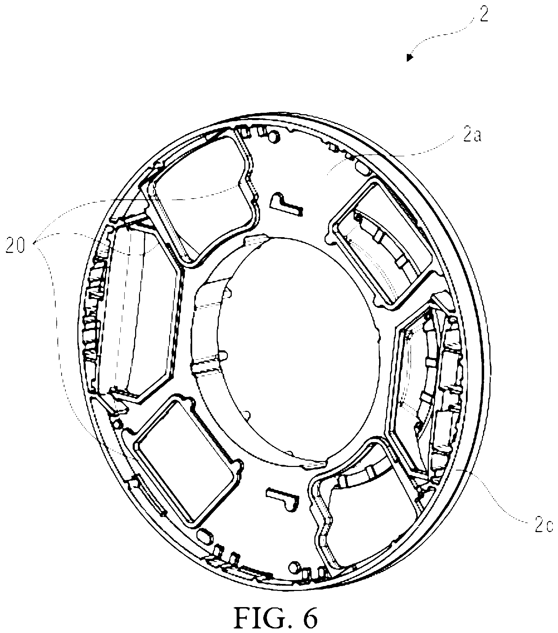

Referring to FIG. 3, the front drum 1 may include at least one front protrusion 10 protruding from the first side surface 1a of the front drum 1. The at least one front protrusion 10 is arranged around and in contact with at least one X-ray tube system of the first X-ray tube system and the second X-ray tube system. Similarly, referring to FIG. 6, the rear drum 2 may include at least one rear protrusion 20 protruding from the first side surface 2a of the rear drum 2. The at least one rear protrusion 20 is arranged around and in contact with at least one set of components of the first set of components and the second set of components. Specifically, at least one of the at least one front protrusion 10 is arranged on a radial outer side of the at least one X-ray tube system; and/or at least one of the at least one rear protrusion 20 is arranged on a radial outer side of the at least one set of components. When the front drum 1 and the rear drum 2 rotate at a high speed, the electrical components mounted thereto bear an enormous centrifugal force. Through the arrangement of the front protrusion 10 and the rear protrusion 20, not only the centrifugal force on the electrical components can be born, but also the rigidity of the casting can be increased. In this way, the centrifugal force is converted to the casting material, thereby significantly improving the safety.

Referring to FIG. 2 to FIG. 4, each of the first tube mounting area 111 and the second tube mounting area 121 may have a through hole, so that the first tube mounted in the first tube mounting area 111 and the second tube mounted in the second tube mounting area 121 pass through the corresponding through holes and are mounted to the front drum 1. In this way, a part of the first tube is located on a first side of the front drum 1, and the other part is located on a second side of the front drum 1, and a part of the second tube is located on the first side of the front drum 1 and the other part is located on the second side of the front drum 1, thereby facilitating the structural balance.

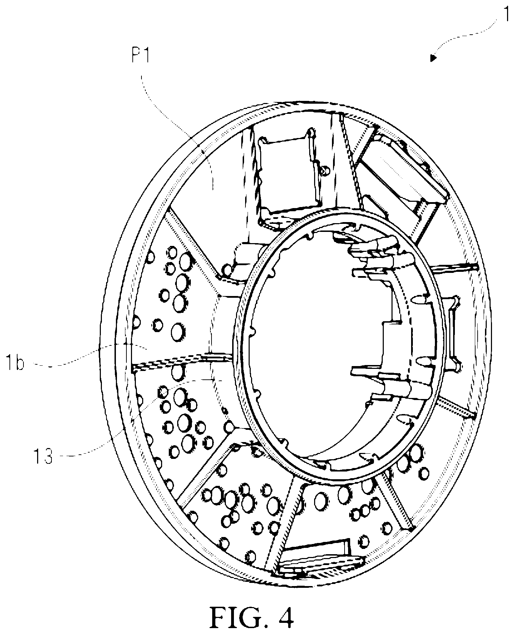

Referring to FIG. 3, a data transmission system may be mounted to a side end surface 1c of the front drum 1 for data transmission with the electrical components. Referring to FIG. 4, a balance weight (not shown) may be mounted at a position P1 on the second side surface 1b of the front drum 1 staggered from the first system mounting area 11 and the second system mounting area 12 located on the first side surface 1a of the front drum 1, thereby facilitating the structural balance.

Referring to FIG. 2 and FIG. 5, one or more grooves G1, G2 may arranged on circumferential surfaces of inner holes of the front drum 1 and the rear drum 2, and the first X-ray tube system and the second X-ray tube system (not shown) may be respectively connected to the first set of components and the second set of components through cables extending through the one or more grooves G1, G2.

Referring to FIG. 5, each of the first component mounting area 21 and the second component mounting area 22 includes a high voltage generator mounting area 211, 221 configured for a high voltage generator to be mounted, a high pressure tank mounting area 212, 222 configured for a high pressure tank to be mounted, and a tube cooling system mounting area 213, 223 configured for a tube cooling system to be mounted, and the first component mounting area 21 and the second component mounting area 22 are arranged symmetrically with respect to a center of the rear drum 2. In this way, self-balance can be realized. Therefore, only a weight distribution of the drum and weight differences between the components need to be balanced and adjusted, thereby reducing the weight of the balance weight and reducing the balance adjustment time.

Referring to FIG. 2 to FIG. 7, due to the high power and the large amount of heat of the electrical components, a heat sink and/or a heat dissipation hole may be arranged on the front drum 1 and/or the rear drum 2, such as a heat dissipation hole in the first detector mounting area 112 or the second detector mounting area 122 extending through the front drum 1. The heat dissipation hole is usually arranged near electrical components on the drum that generate a large amount of heat. In addition, the drum casting may be designed as a thin-walled frame structure to facilitate heat dissipation.

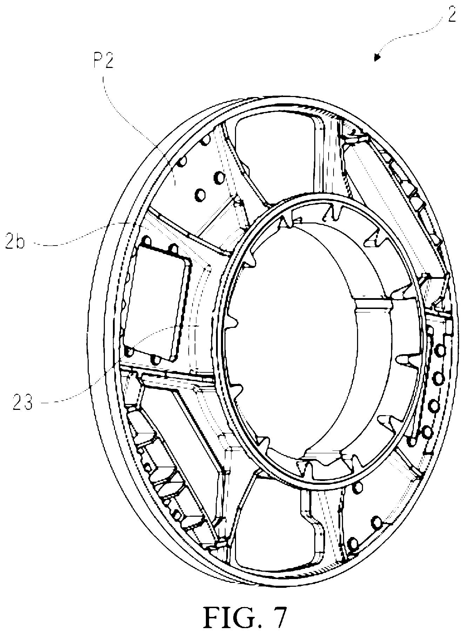

Referring to FIG. 6, a slip ring system may be mounted to a side end surface 2c of the rear drum 2 for transmitting power and signals. Referring to FIG. 7, a balance weight (not shown) is mounted at a position (P2) on the second side surface 2b of the rear drum 2 staggered from the first component mounting area 21 and the second component mounting area 22 located on the first side surface 2a of the rear drum 2.

Referring to FIG. 4 and FIG. 7, the front drum 1 may include a plurality of support ribs connected between the second side surface 1b of the front drum 1 and the front flange 13 and spaced apart in a circumferential direction. Similarly, the rear drum 2 may also include a plurality of support ribs connected between the second side surface 2b of the rear drum 2 and the rear flange 23 and spaced apart in the circumferential direction. The arrangement of the support ribs increases the structural stability of the front drum 1 and the rear drum.

A dual source CT machine of the invention includes the drum in the above embodiment. The front drum and the rear drum of the drum are mounted to an outer rim of a bearing of the CT machine, and an inner rim of the bearing is mounted to a gantry of the CT machine.

Through the drum of the invention including the front drum 1 and the rear drum 2, the scan time of the CT machine can be reduced, the scan efficiency can be improved, and clearer scanned images can be obtained. The drum of the invention has a light weight and low costs, is integrated with many functions, and has a more optimized structure.

## Claims

1. A drum for a dual source CT machine, the drum (100) being mounted to an outer rim of a bearing, an inner rim of the bearing being mounted to a gantry of the dual source CT machine, and the drum (100) comprising:
a ring-shaped front drum (1), wherein the ring-shaped front drum (1) has a first side surface (1a) and a second side surface (1b) opposite to each other, a first system mounting area (11) configured for a first X-ray tube system to be mounted and a second system mounting area (12) configured for a second X-ray tube system to be mounted are arranged on the first side surface (1a) of the front drum (1), and a front flange (13) extending in an axial direction from an inner ring edge of the front drum (1) is formed on the second side surface (1b) of the front drum (1); and
a ring-shaped rear drum (2), wherein the rear drum (2) has a first side surface (2a) and a second side surface (2b) opposite to each other, a first component mounting area (21) configured for a first set of components matching the first X-ray tube system to be mounted and a second component mounting area (22) configured for a second set of components matching the second X-ray tube system to be mounted are arranged on the first side surface (2a) of the rear drum (2), and a rear flange (23) extending in the axial direction from an inner ring edge of the rear drum (2) is formed on the second side surface (2b) of the rear drum (2); and
the front drum (1) and the rear drum (2) are arranged coaxially, the front flange (13) and the rear flange (23) face each other, the front drum (1) is connected to the bearing through the front flange (13), and the rear drum (2) is connected to the bearing through the rear flange (23).

2. The drum for a dual source CT machine according to claim 1, wherein the first system mounting area (11) comprises a first tube mounting area (111) configured for a first tube to be mounted and a first detector mounting area (112) configured for a first detector to be mounted, and a first X-ray path (R1) is formed between the first tube and the first detector; the second system mounting area (12) comprises a second tube mounting area (121) configured for a second tube to be mounted and a second detector mounting area (122) configured for a second detector to be mounted, and a second X-ray path (R2) is formed between the second tube and the second detector; and the first X-ray path (R1) and the second X-ray path (R2) are both perpendicular to the axial direction, and an included angle (α) between the first X-ray path (R1) and the second X-ray path (R2) is between 45 degrees and 135 degrees.

3. The drum for a dual source CT machine according to claim 1 or 2, wherein
the front drum (1) comprises at least one front protrusion (10) protruding from the first side surface (1a)of the front drum (1), and the at least one front protrusion (10) is arranged around and in contact with at least one X-ray tube system of the first X-ray tube system and the second X-ray tube system; and/or
the rear drum (2) comprises at least one rear protrusion (20) protruding from the first side surface (2a) of the rear drum (2), and the at least one rear protrusion (20) is arranged around and in contact with at least one set of components of the first set of components and the second set of components.

4. The drum for a dual source CT machine according to claim 3, wherein
at least one of the at least one front protrusion (10) is arranged on a radial outer side of the at least one X-ray tube system; and/or
at least one of the at least one rear protrusion (20) is arranged on a radial outer side of the at least one set of components.

5. The drum for a dual source CT machine according to claim 2, wherein each of the first tube mounting area (111) and the second tube mounting area (121) has a through hole, so that the first tube mounted in the first tube mounting area (111) and the second tube mounted in the second tube mounting area (121) pass through the corresponding through holes and are mounted to the front drum (1).

6. The drum for a dual source CT machine according to claim 1, wherein a balance weight is mounted at a position on the second side surface (1b) of the front drum (1) staggered from the first system mounting area (11) and the second system mounting area (12) located on the first side surface (1a) of the front drum (1).

7. The drum for a dual source CT machine according to claim 1, wherein one or more grooves (G1, G2) are arranged on circumferential surfaces of inner holes of the front drum (1) and the rear drum (2), and the first X-ray tube system and the second X-ray tube system are respectively connected to the first set of components and the second set of components through cables extending through the one or more grooves (G1, G2).

8. The drum for a dual source CT machine according to claim 1, wherein each of the first component mounting area (21) and the second component mounting area (22) comprises a high voltage generator mounting area (211, 221) configured for a high voltage generator to be mounted, a high pressure tank mounting area (212, 222) configured for a high pressure tank to be mounted, and a tube cooling system mounting area (213, 223) configured for a tube cooling system to be mounted, and the first component mounting area (21) and the second component mounting area (22) are arranged symmetrically with respect to a center of the rear drum (2).

9. The drum for a dual source CT machine according to claim 1, wherein a balance weight is mounted at a position on the second side surface (2b) of the rear drum (2) staggered from the first component mounting area (21) and the second component mounting area (22) located on the first side surface (2a) of the rear drum (2).

10. A dual source CT machine, comprising the drum for a dual source CT machine according to any of claims 1 to 9.

## Patentansprüche

1. Trommel für ein Dual-Source-CT-Gerät, wobei die Trommel (100) an einem äußeren Rand eines Lagers montiert ist, ein innerer Rand des Lagers an einer Gantry der Dual-Source-CT-Gerät montiert ist und die Trommel (100) Folgendes umfasst:
eine ringförmige vordere Trommel (1), wobei die ringförmige vordere Trommel (1) eine erste Seitenfläche (1a) und eine zweite Seitenfläche (1b) aufweist, die einander gegenüber liegen, wobei ein erster Systemmontagebereich (11), der für die Montage eines ersten Röntgenröhrensystem ausgestaltet ist, und ein zweiter Systemmontagebereich (12), der für die Montage eines zweiten Röntgenröhrensystem ausgestaltet ist, auf der ersten Seitenfläche (1a) der vorderen Trommel (1) angeordnet sind, und ein vorderer Flansch (13), der sich von einer inneren Ringkante der vorderen Trommel (1) in einer axialen Richtung erstreckt, an der zweiten Seitenfläche (1b) der vorderen Trommel (1) ausgebildet ist; und
eine ringförmige hintere Trommel (2), wobei die hintere Trommel (2) eine erste Seitenfläche (2a) und eine zweite Seitenfläche (2b) aufweist, die einander gegenüber liegen, wobei ein erster Komponentenmontagebereich (21), der für einen ersten Satz von Komponenten ausgestaltet ist, der zu dem zu montierenden ersten Röntgenröhrensystem passt, und ein zweiter Komponentenmontagebereich (22), der für einen zweiten Satz von Komponenten ausgestaltet ist, der zu dem zu montierenden zweiten Röntgenröhrensystem passt, auf der ersten Seitenfläche (2a) der hinteren Trommel (2) angeordnet sind, und ein hinterer Flansch (23), der sich in axialer Richtung von einer inneren Ringkante der hinteren Trommel (2) erstreckt, an der zweiten Seitenfläche (2b) der hinteren Trommel (2) ausgebildet ist; und
die vordere Trommel (1) und die hintere Trommel (2) koaxial angeordnet sind, der vordere Flansch (13) und der hintere Flansch (23) zueinander weisen, die vordere Trommel (1) über den vorderen Flansch (13) mit dem Lager verbunden ist, und die hintere Trommel (2) über den hinteren Flansch (23) mit dem Lager verbunden ist.

2. Trommel für ein Dual-Source-CT-Gerät nach Anspruch 1, wobei der erste Systemmontagebereich (11) einen ersten Röhrenmontagebereich (111), der für die Montage einer ersten Röhre ausgestaltet ist, und einen ersten Detektormontagebereich (112), der für die Montage eines ersten Detektors ausgestaltet ist, umfasst, und ein erster Röntgenpfad (R1) zwischen der ersten Röhre und dem ersten Detektor ausgebildet ist; der zweite Systemmontagebereich (12) einen zweiten Röhrenmontagebereich (121), der für die Montage einer zweiten Röhre ausgestaltet ist, und einen zweiten Detektormontagebereich (122), der für die Montage eines zweiten Detektors ausgestaltet ist, umfasst, und ein zweiter Röntgenpfad (R2) zwischen der zweiten Röhre und dem zweiten Detektor ausgebildet ist; und der erste Röntgenpfad (R1) und der zweite Röntgenpfad (R2) beide rechtwinklig zu der axialen Richtung sind und ein eingeschlossener Winkel (α) zwischen dem ersten Röntgenpfad (R1) und dem zweiten Röntgenpfad (R2) zwischen 45 Grad und 135 Grad liegt.

3. Trommel für ein Dual-Source-CT-Gerät nach Anspruch 1 oder 2, wobei
die vordere Trommel (1) mindestens einen vorderen Vorsprung (10) umfasst, der von der ersten Seitenfläche (1a) der vorderen Trommel (1) vorsteht, und der mindestens eine vordere Vorsprung (10) um und in Kontakt mit mindestens einem Röntgenröhrensystem des ersten Röntgenröhrensystems und des zweiten Röntgenröhrensystems angeordnet ist; und/oder
die hintere Trommel (2) mindestens einen hinteren Vorsprung (20) umfasst, der von der ersten Seitenfläche (2a) der hinteren Trommel (2) vorsteht, und der mindestens eine hintere Vorsprung (20) um und in Kontakt mit mindestens einem Satz von Komponenten des ersten Satzes von Komponenten und des zweiten Satzes von Komponenten angeordnet ist.

4. Trommel für ein Dual-Source-CT-Gerät nach Anspruch 3, wobei
mindestens einer des mindestens einen vorderen Vorsprungs (10) an einer radialen Außenseite des mindestens einen Röntgenröhrensystems angeordnet ist; und/oder
mindestens einer des mindestens einen hinteren Vorsprungs (20) auf einer radialen Außenseite des mindestens einen Satzes von Komponenten angeordnet ist.

5. Trommel für ein Dual-Source-CT-Gerät nach Anspruch 2, wobei jeder von dem ersten Röhrenmontagebereich (111) und dem zweiten Röhrenmontagebereich (121) ein Durchgangsloch aufweist, so dass die erste Röhre, die in dem ersten Röhrenmontagebereich (111) montiert ist, und die zweite Röhre, die in dem zweiten Röhrenmontagebereich (121) montiert ist, durch die entsprechenden Durchgangslöcher verlaufen und an der vorderen Trommel (1) montiert sind.

6. Trommel für ein Dual-Source-CT-Gerät nach Anspruch 1, wobei ein Ausgleichsgewicht an einer Position auf der zweiten Seitenfläche (1b) der vorderen Trommel (1) montiert ist, die zu dem ersten Systemmontagebereich (11) und dem zweiten Systemmontagebereich (12) gestaffelt ist, die sich auf der ersten Seitenfläche (1a) der vorderen Trommel (1) befinden.

7. Trommel für ein Dual-Source-CT-Gerät nach Anspruch 1, wobei eine oder mehrere Nuten (G1, G2) auf Umfangsflächen von Innenlöchern der vorderen Trommel (1) und der hinteren Trommel (2) angeordnet ist/sind, und das erste Röntgenröhrensystem und das zweite Röntgenröhrensystem jeweils mit dem ersten Satz von Komponenten und dem zweiten Satz von Komponenten durch Kabel verbunden sind, die sich durch die eine oder die mehreren Nuten (G1, G2) erstrecken.

8. Trommel für ein Dual-Source-CT-Gerät nach Anspruch 1, wobei jeder von dem ersten Komponentenmontagebereich (21) und dem zweiten Komponentenmontagebereich (22) einen Hochspannungsgeneratormontagebereich (211, 221), der für die Montage eines Hochspannungsgenerators ausgestaltet ist, einen Hochdrucktankmontagebereich (212, 222), der für die Montage eines Hochdrucktanks ausgestaltet ist, und einen Röhrenkühlsystemmontagebereich (213, 223) umfasst, der für die Montage eines Röhrenkühlsystems ausgestaltet ist, und der erste Komponentenmontagebereich (21) und der zweite Komponentenmontagebereich (22) symmetrisch in Bezug auf eine Mitte der hinteren Trommel (2) angeordnet sind.

9. Trommel für ein Dual-Source-CT-Gerät nach Anspruch 1, wobei ein Ausgleichsgewicht an einer Position auf der zweiten Seitenfläche (2b) der hinteren Trommel (2) montiert ist, die zu dem ersten Komponentenmontagebereich (21) und dem zweiten Komponentenmontagebereich (22) gestaffelt ist, die sich auf der ersten Seitenfläche (2a) der hinteren Trommel (2) befinden.

10. Dual-Source-CT-Gerät, das die Trommel für ein Dual-Source-CT-Gerät nach einem der Ansprüche 1 bis 9 umfasst.

## Revendications

1. Tambour pour une machine de tomodensitométrie à double source, le tambour (100) étant monté sur un bord extérieur d'un palier, un bord intérieur du palier étant monté sur un portique de la machine de tomodensitométrie à double source, et le tambour (100) comprenant :
un tambour avant en forme d'anneau (1), le tambour avant en forme d'anneau (1) ayant une première surface latérale (1a) et une seconde surface latérale (1b) opposées l'une à l'autre, une première zone de montage de système (11) configurée pour un premier système de tube à rayons X et une seconde zone de montage de système (12) configurée pour un second système de tube à rayons X étant agencées sur la première surface latérale (1a) du tambour avant (1), et une bride avant (13) s'étendant dans une direction axiale depuis un bord annulaire interne du tambour avant (1) étant formée sur la seconde surface latérale (1b) du tambour avant (1) ; et
un tambour arrière en forme d'anneau (2), le tambour arrière (2) ayant une première surface latérale (2a) et une seconde surface latérale (2b) opposées l'une à l'autre, une première zone de montage de composants (21) configurée pour un premier ensemble de composants correspondant au premier système de tube à rayons X et une seconde zone de montage de composants (22) configurée pour un second ensemble de composants correspondant au second système de tube à rayons X étant agencées sur la première surface latérale (2a) du tambour arrière (2), et une bride arrière (23) s'étendant dans la direction axiale depuis un bord annulaire interne du tambour arrière (2) étant formée sur la seconde surface latérale (2b) du tambour arrière (2) ; et
le tambour avant (1) et le tambour arrière (2) étant agencés de manière coaxiale, la bride avant (13) et la bride arrière (23) étant orientées l'une vers l'autre, le tambour avant (1) étant relié au palier par l'intermédiaire de la bride avant (13), et le tambour arrière (2) étant relié au palier par l'intermédiaire de la bride arrière (23).

2. Tambour pour une machine de tomodensitométrie à double source selon la revendication 1, la première zone de montage de système (11) comprenant une première zone de montage de tube (111) configurée pour un premier tube et une première zone de montage de détecteur (112) configurée pour un premier détecteur, un premier trajet de rayons X (R1) étant formé entre le premier tube et le premier détecteur ; la seconde zone de montage de système (12) comprenant une seconde zone de montage de tube (121) configurée pour un second tube et une seconde zone de montage de détecteur (122) configurée pour un second détecteur, un second trajet de rayons X (R2) étant formé entre le second tube et le second détecteur ; et le premier trajet de rayons X (R1) et le second trajet de rayons X (R2) étant tous deux perpendiculaires à la direction axiale, et un angle inclus (α) entre le premier trajet de rayons X (R1) et le second trajet de rayons X (R2) étant compris entre 45 degrés et 135 degrés.

3. Tambour pour une machine de tomodensitométrie à double source selon la revendication 1 ou 2,
le tambour avant (1) comprenant au moins une saillie avant (10) faisant saillie depuis la première surface latérale (1a) du tambour avant (1), et l'au moins une saillie avant (10) étant agencée autour d'au moins un système de tube à rayons X parmi le premier système de tube à rayons X et le second système de tube à rayons X et en contact avec celui-ci ; et/ou
le tambour arrière (2) comprenant au moins une saillie arrière (20) faisant saillie depuis la première surface latérale (2a) du tambour arrière (2), et l'au moins une saillie arrière (20) étant agencée autour d'au moins un ensemble de composants parmi le premier ensemble de composants et le second ensemble de composants et en contact avec celui-ci.

4. Tambour pour une machine de tomodensitométrie à double source selon la revendication 3,
au moins l'une de l'au moins une saillie avant (10) étant agencée sur un côté radialement externe de l'au moins un système de tube à rayons X ; et/ou
au moins l'une de l'au moins une saillie arrière (20) étant agencée sur un côté radialement externe de l'au moins un ensemble de composants.

5. Tambour pour une machine de tomodensitométrie à double source selon la revendication 2, la première zone de montage de tube (111) et la seconde zone de montage de tube (121) comportant chacune un trou traversant, de sorte que le premier tube monté dans la première zone de montage de tube (111) et le second tube monté dans la seconde zone de montage de tube (121) traversent les trous traversants correspondants et sont montés sur le tambour avant (1).

6. Tambour pour une machine de tomodensitométrie à double source selon la revendication 1, un contrepoids étant monté en une position sur la seconde surface latérale (1b) du tambour avant (1) décalée par rapport à la première zone de montage de système (11) et à la seconde zone de montage de système (12) situées sur la première surface latérale (1a) du tambour avant (1).

7. Tambour pour une machine de tomodensitométrie à double source selon la revendication 1, une ou plusieurs rainures (G1, G2) étant agencées sur des surfaces circonférentielles de trous internes du tambour avant (1) et du tambour arrière (2), et le premier système de tube à rayons X et le second système de tube à rayons X étant respectivement reliés au premier ensemble de composants et au second ensemble de composants par l'intermédiaire de câbles s'étendant à travers la ou les rainures (G1, G2).

8. Tambour pour une machine de tomodensitométrie à double source selon la revendication 1, la première zone de montage de composants (21) et la seconde zone de montage de composants (22) comprenant chacune une zone de montage de générateur haute tension (211, 221) configurée pour un générateur haute tension, une zone de montage de réservoir haute pression (212, 222) étant configurée pour un réservoir haute pression, et une zone de montage de système de refroidissement de tube (213, 223) étant configurée pour un système de refroidissement de tube, et la première zone de montage de composants (21) et la seconde zone de montage de composants (22) étant agencées symétriquement par rapport à un centre du tambour arrière (2).

9. Tambour pour une machine de tomodensitométrie à double source selon la revendication 1, un contrepoids étant monté en une position sur la seconde surface latérale (2b) du tambour arrière (2) décalée par rapport à la première zone de montage de composants (21) et à la seconde zone de montage de composants (22) situées sur la première surface latérale (2a) du tambour arrière (2).

10. Machine de tomodensitométrie à double source, comprenant le tambour pour une machine de tomodensitométrie à double source selon l'une quelconque des revendications 1 à 9.
